## Europäisches Patentamt
## European Patent Office
## Office européen des brevets

(19)

(11) Veröffentlichungsnummer : **0 047 339**
**B1**

(12)

# EUROPÄISCHE PATENTSCHRIFT

(45) Veröffentlichungstag der Patentschrift :
08.02.84

(21) Anmeldenummer : 80107390.9

(22) Anmeldetag : 26.11.80

(51) Int. Cl.³ : **A 61 K 31/395**, A 61 K   9/06 //
(A61K31/395, 31/22)

(54) Arzneimittel zur lokalen Behandlung des Glaukoms.

(30) Priorität : 09.09.80 DE 3033898

(43) Veröffentlichungstag der Anmeldung :
17.03.82 Patentblatt 82/11

(45) Bekanntmachung des Hinweises auf die Patenterteilung : 08.02.84 Patentblatt 84/06

(84) Benannte Vertragsstaaten :
AT CH DE FR GB IT LI SE

(56) Entgegenhaltungen :
US-A- 3 839 584
UNLISTED DRUGS, April 1978, Chatham, New Jersey, U.S.A. "Ganda"
UNLISTED DRUGS, Band 32, Mai 1980, Seite 75c,
Chatham, New Jersey, U.S.A. "Suprexon"

(73) Patentinhaber : **Dr. Thilo & Co. GmbH**
**Rudolf-Diesel-Ring 21**
**D-8029 Sauerlach (DE)**

(72) Erfinder : **Ober, Manuel, Dr.**
**Amselweg 7**
**D-8521 Uttenreuth (DE)**
Erfinder : **Scharrer, Armin, Dr.**
**Sieglitzhoferstrasse 42**
**D-8520 Erlangen (DE)**

(74) ·Vertreter : **von Füner, Alexander, Dr. et al**
**Patentanwälte v. Füner, Ebbinghaus, Finck Mariahilf-**
**platz 2 & 3**
**D-8000 München 90 (DE)**

Jouve, 18, rue St-Denis, 75001 Paris, France

## Arzneimittel zur lokalen Behandlung des Glaukoms

Die Erfindung betrifft ein Arzneimittel zur Anwendung am Auge in Form von Augentropfen, Augensalbe, Augengel, Augeninsert oder Lyophilisat zur Bereitung von Augentropfen, das neben ± Epinephrin-O$^1$,O$^2$-dipivalat in therapeutisch üblichen Dosierungen (0,05-0,2 %) als zweiten Wirkstoff Guanethidin in Konzentrationen enthält, die weit unter der bisher üblichen Dosierung liegen, nämlich bei nur 0,05-1,0 %.

Klassische Arzneimittel zur Behandlung des Glaukoms, einer krankhaften Steigerung des Augeninnendruckes (IOP) mit schädigender Einwirkung auf Schnerv und Netzhat, sind Adrenalin-Epinephrin-) und Guanethidin-Augentropfen, im Handel u. a. unter den Warenzeichen EPPY-Augentropfen (1 % Adrenalin), GLAUCON (2 % Adrenalin), ISOPTO EPINAL-Augentropfen (1 % Adrenalin) bzw. ISMELIN-Augentropfen (5 % Guanethidinsulfat).

Da durch Guanethidin die Adrenalin-Wirkung verstärkt wird, lag es nahe, beide Wirkstoffe gleichzeitig am glaukomatösen Auge anzuwenden. Entsprechende Kombinationspräparate sind in England unter der Bezeichnung GANDA (R) 3 + 0,5 bzw. 5 + 0,5 bzw. 5 + 1 auf dem Markt, wobei die erste Zahl die Konzentration an Guanethidinsulfat (3 bzw. 5 %), die zweite Zahl die Konzentration an Adrenalin (0,5 bzw. 1 %) angibt.

Es gibt jedoch viele Nachteile für eine hochdosierte lokale Guanethidin-Therapie. Bekannt ist die schlechte Schleimhautverträglichkeit 3 bis 5 %-iger Guanethidinsulfat-Augentropfen (Brennen, Rötung der Augen infolge von Bindehautreizung, Lokalanästhesie), die teils wirkstoffbedingt ist, teils durch den unphysiologisch hohen osmotischen Druck dieser Augentropfen hervorgerufen wird. Manche Patienten klagen über eine Beeinträchtigung des Geschmacks.

Vor allem aber besteht auch die Gefahr systemischer Nebenwirkungen bei lokaler Guanethidin-Anwendung. Mit 2 × täglich 1 Tropfen Guanethidinsulfat 3 % bzw. 5 % pro Auge werden dem Organismus täglich 6 mg bzw. 10 mg des Wirkstoffes zugeführt. Da Guanethidinsulfat oral als hochwirksames Antihypertonikum in Dosen von 10 bis 30 mg pro Tag eingesetzt wird, sind bei Anwendung der bisher üblichen guanethidinhaltigen Augentropfen unerwünschte Nebenwirkungen, wie orthostatische Regulationsstörungen, Adynamie und Miktionsstörungen zu erwarten (siehe ROTE LISTE 1980, Nebenwirkungen G 35 = Guanethidin). Durch den möglichen Blutdruckabfall wird sogar die Netzhautdurchblutung verschlechtert, wodurch — trotz normalisierten IOP's — die Gefahr eines Sehverlustes entsteht.

Überraschenderweise wurde nun gefunden, daß die Guanethidin-Konzentration um 67 bis 99 % reduziert werden kann, wenn der Wirkstoff anstatt mit Adrenalin mit der Adrenalin-Prodrug Dipivefrin kombiniert wird.

Dipivefrin, auch (+/−)-Epinephrin-O$^1$,O$^2$-dipivalat genannt, ist Bestandteil der deutschen Arzneimittel D-EPIFRIN 0,1 % (R) und GLAUCOTHIL 0,1 % (R) und besitzt dank seiner besseren Cornea-Gängigkeit eine ca. zehnfach stärkere Wirkung am Auge als die Muttersubstanz Adrenalin.

Wird das Glaukomauge mit 0,05-0,2 %igen ± Epinephrin-O$^1$,O$^2$-dipivalathydrochlorid-Augentropfen (entsprechend in der Wirkung 0,5 bis 2 %igen Adrenalin-Augentropfen) behandelt, so zeigt bereits die zusätzliche lokale Anwendung von 0,05 bis 1,0 % Guanethidinsulfat eine deutliche Wirkungsverstärkung.

Dies ist ein absolut überraschendes Phänomen, für das bisher noch keine pharmakologische Erklärung gefunden werden konnte. Nach dem derzeitigen Wissensstand wäre ja zu erwarten, daß zur Erzielung einer Wirkungssteigerung von Adrenalin bzw. der Wirkstoff Guanethidinsulfat in 3 bis 5 %iger, also drei- bis hundertfach höherer Konzentration angewendet werden muß.

Der Gegenstand der Erfindung ist aus den vorstehenden Ansprüchen ersichtlich.

Die Erfindung betrifft ein Arzneimittel zur lokalen Behandlung des Glaukoms in Form von Augentropfen, Lyophilisat zur Bereitung von Augentropfen, Augensalbe, Augengel und Augeninsert, daß dadurch gekennzeichnet ist, daß es ein ± Epinephrin-O$^1$,O$^2$-dipivalatsalz und ein Guanethidinsalz enthält.

Nach einer bevorzugten Ausführungsform liegt das ± Epinephrin-O$^1$,O$^2$-dipivalatsalz in einer Konzentration von 0,05 bis 0,2 % als Salz der nachfolgenden Säuren vor : Salzsäure, Salpetersäure, Schwefelsäure, Borsäure, Phosphorsäure, Zitronensäure, Weinsäure, Essigsäure, Propionsäure oder Bernsteinsäure.

Es ist ferner bevorzugt, daß das Guanethidin in der Konzentration von 0,05 bis 1,0 % als Salz der nachfolgenden Säuren vorliegt : Schwefelsäure, Salzsäure, Salpetersäure, Borsäure, Phosphorsäure, Zitronensäure, Weinsäure, Essigsäure, Propionsäure oder Bernsteinsäure.

Besonders bevorzugt als Bestandteile des erfindungsgemäßen Arzneimittels sind das ± Epinephrin-O$^1$,O$^2$-dipivalathydrochlorid und das Guanethidinsulfat.

Der Gegenstand der Erfindung ist in der bevorzugten Ausführungsform die gleichzeitige Anwendung von ± Epinephrin-O$^1$,O$^2$-dipivalathydrochlorid in therapeutisch üblichen Dosierungen (0,05 bis 0,2 %) und von Guanethidinsulfat in Konzentrationen, die weit unter der bisher üblichen Dosierung liegen, nämlich bei nur 0,05 bis 1,0 %.

Eine solche Kombination weist therapeutische Vorteile auf :

1) Ausgezeichnete Schleimhautverträglichkeit, da die Arzneimittel mit einem der Tränenflüssigkeit entsprechenden osmotischen Druck hergestellt werden können und die bindehautreizende Grenzkonzentration für Guanethidin weit unterschritten wird.

2) Fehlende Nebenwirkungen, da die Adrenalin-Prodrug ± Epinephrin-O$^1$,O$^2$-dipivalat wesentlich niedriger dosiert werden kann als die Muttersubstanz Adrenalin und da — was noch wichtiger ist — dem Organismus Guanethidin zugeführt wird in Mengen, die weit unter der systemisch-therapeutisch üblichen Dosierung liegen. Bei 2 × täglich 1 Tropfen Guanethidinsulfat 0,05 % bzw. 1 % pro Auge wird der Körper nur mit 0,1 bzw. 2 mg/Tag belastet.

Für die Kombination ± Epinephrin-O$^1$,O$^2$-dipivalathydrochlorid 0,05 bis 0,2 % + Guanethidinsulfat 0,05 bis 1,0 % kommen alle für die Oculo-Therapie üblichen Arzneiformen in Frage, insbesondere Augentropfen, Lyophilisat zur Bereitung von Augentropfen, Augensalben, Augengele und Augeninserts, die letzteren beiden Zubereitungen auch mit verzögerter Wirkstoffabgabe. Die beiden Wirkstoffe können darin als Salze verschiedener anorganischer oder organischer Säuren vorliegen : ± Epinephrin-O$^1$,O$^2$-dipivalat insbesondere als Chlorid, Nitrat, Sulfat, Borat, Phosphat, Citrat, Tartrat, Acetat, Propionat und Succinat ; Guanethidin insbesondere als Sulfat, Chlorid, Nitrat, Borat, Phosphat, Citrat, Tartrat, Acetat, Propionat und Succinat, auch in Form der Hemisalze.

Als Hilfsstoffe können alle für Augenarzneien üblichen Verbindungen enthalten sein :

Zum Angleichen des osmotischen Druckes an jenen der Tränenflüssigkeit : Chloride, Sulfate, Phosphate, Borate, Nitrate, Citrate, Tartrate, Acetate, Propionate und Succinate in Form ihrer wasserlöslichen Salze.

Zur Stabilisierung der Wirkstoffe :

Physiologisch unbedenkliche Antioxydantien wie Natrium- und Kaliumsulfite, thiolgruppenhaltige Verbindungen, Ascorbinsäure und Komplexierende Stoffe wie die Salze der Äthylendiamintetraessigsäure, der Diäthylentriaminpentaessigsäure, der Hydroxyäthylendiamintriessigsäure.

Zur Konsistenzgebung (viskose Lösung, Gel, Salbe, Insert) und als Filmbildner zur Verbesserung der Verträglichkeit und der Wirkung :

Celluloseäther wie Äthyl-, Methyl-, Hydroxyäthyl- und Hydroxypropylmethylcellulose ; Carboxymethylcellulose ; Polyvinylpyrrolidone ; Polyvinylalkohole ; Polyäthylenoxide mit MG 100 000 ; Polyäthylenglykole mit MG 400 ; Salze der Polyacrylsäuren ; Dextrane ; Alginate ; Pektinate ; Polyoxyäthylenpolyoxypropylen-Blockpolymerisate ; gereinigte feste und flüssige Kohlenwasserstoffe ; Emulgatoren wie Sorbitanfettsäureester, äthoxylierte Sorbitanfettsäureester, Wollwachsalkohole, Lecithine, Miranole.

Zur Konservierung der Zubereitungen :

Phenylquecksilbersalze, anionaktive Organoquecksilberverbindungen wie Thiomersal, quaternäre Ammoniumverbindungen wie Benzalkoniumchlorid, Chlorhexidinsalze, Chlorobutanol, Sorbinsäure, Parabene und Phenyläthylalkohol.


Beispiel 1


Ein Kollektiv von 30 Patienten mit Glaucoma simplex oder okulärer Hypertension wurde im Verlauf von 1 bis 4 Wochen mit ± Epinephrin-O$^1$,O$^2$-dipivalathydrochlorid 0,1 %-Augentropfen auf einen niedrigeren IOP eingestellt. Nach dem Erreichen konstanter IOP-Werte wurden die Patienten in drei Gruppen zu jeweils 10 Patienten eingeteilt und zusätzlich mit Guanethidinsulfat-Augentropfen verschiedener Konzentration behandelt.

Gruppe 1 (durchschnittlicher IOP 3 600 Pa (27 mmHg)) wurde weiterbehandelt mit Epinephrin-O$^1$,O$^2$-dipivalathydrochlorid 0,1 % und zusätzlich Guanethidinsulfat (GMS) 0,25 %.

Bereits 6 bis 12 Stunden nach der ersten Anwendung dieser Kombination sank der IOP signifikant bei allen Patienten. Im Verlauf einer Woche pendelte sich der durchschnittliche IOP auf 3 066 Pa (23 mmHg) ein. Die durch 0,25 % GMS bedingte zusätzliche Drucksenkung betrug also 533 Pa (4 mmHg), entsprechend 15 % des Ausgangswertes.

Gruppe 2 (durchschnittlicher IOP 3 466 Pa (26 mmHg)) wurde nach der initialen Epinephrin-O$^1$,O$^2$-dipivalathydrochlorid 0,1 %-Therapie weiterbehandelt mit Epinephrin-O$^1$,O$^2$-dipivalathydrochlorid 0,1 % + GMS 0,5 %.

Nach einwöchiger Behandlung war ein konstanter durchschnittlicher IOP von 2 800 Pa (21 mmHg) erreicht. Die durch 0,5 % GMS bedingte zusätzliche Drucksenkung betrug also 667 Pa (5 mmHg), entsprechend 19 % des Ausgangswertes.

Gruppe 3 (durchschnittlicher IOP nach der anfänglichen Monotherapie mit 0,1 % Epinephrin-O$^1$,O$^2$-dipivalathydrochlorid 3 600 Pa (27 mmHg) wurde weiterbehandelt mit Epinephrin-O$^1$,O$^2$-dipivalathydrochlorid 0,1 % + GMS 1 %.

Nach einwöchiger Behandlung betrug der durchschnittliche konstante IOP 2 886 Pa (21,5 mmHg). Die durch 1 % GMS bedingte zusätzliche Drucksenkung betrug also 733 Pa (5,5 mmHg), entsprechend 20 % des Ausgangswertes.

3

### Ergebnisse

Aus dieser Untersuchungs-Serie ist zu ersehen, daß die kombinierte lokale Epinephrin-$O^1,O^2$-dipivalathydrochlorid/GMS-Therapie gegenüber der Monotherapie mit DPE Vorteile bietet, nämlich zu einer weiteren Senkung des Augeninnendruckes führt.

Die Kombination mit 0,5 % GMS erweist sich dabei der Kombination mit 0,25 % GMS überlegen. Zwischen den 0,5 % GMS und 1 % GMS enthaltenden Kombinationen besteht jedoch kein signifikanter Unterschied.

Dies bedeutet, daß für die ophthalmologische Praxis normalerweise die Kombinationen Epinephrin-$O^1,O^2$-dipivalathydrochlorid 0,05 bis 0,2 % + GMS 0,25 % und Epinephrin-$O^1,O^2$-dipivalathydrochlorid 0,05 bis 0,2 % + GMS 0,5 % ausreichen werden. Nur in Problemfällen (besonders stark erhöhte IOP-Ausgangswerte, therapieresistente Patienten) wird auf die Kombination Epinephrin-$O^1,O^2$-dipivalathydrochlorid 0,05 bis 0,2 % + GMS 1 % übergegangen.

Bei der augenärztlichen Untersuchung der insgesamt 60 Augen wurde objektiv eine kaum wahrnehmbare passagere konjunktivale Hyperämie gesehen, wobei hinsichtlich der Stärke kein Unterschied zwischen den drei GMS-Konzentrationen festgestellt werden konnte.

Alle Patienten, auch jene der Gruppe 3 (höchste GMS-Konzentration) beurteilten subjektiv die Verträglichkeit der Epinephrin-$O^1,O^2$-dipivalathydrochlorid/GMS-Augentropfen als genauso gut wie bei der Erstbehandlung mit Epinephrin-$O^1,O^2$-dipivalathydrochlorid 0,1 % allein.

Ein Einfluß auf den Pupillendurchmesser war nach zusätzlicher Gabe von GMS 0,25 bis 1 % ebensowenig nachweisbar wie ein Einfluß auf die Refraktion.

Bei keinem der Patienten war der Geschmack beeinträchtigt. In Gruppe 2 befanden sich drei, in Gruppe 3 vier Hypertoniker, deren Blutdruck durch übliche Antihypertonika eingestellt war. Regelmäßige Kontrollen ergaben, daß der Blutdruck im Verlauf der lokalen Kombinationstherapie mit DPE 0,1 % + GMS 0,5 % bzw. Epinephrin-$O^1,O^2$-dipivalathydrochlorid 0,1 % + GMS 1 % nicht weiter sank.

### Beispiel 2

Versuchsweise wurde die Patienten-Gruppe 2 aus dem Beispiel 1 im Anschluß an die Kombinationstherapie Epinephrin-$O^1,O^2$-dipivalathydrochlorid 0,1 % + GMS 0,5 % weiterbehandelt mit Adrenalin 1 % + GMS 0,5 %.

Nach einwöchiger Behandlung war der IOP in allen Fällen wieder angestiegen und hielt sich bei einem durchschnittlichen konstanten Wert von 3 733 Pa (28 mmHg), bei einem Wert also, der auch durch die alleinige Therapie mit Epinephrin-$O^1,O^2$-dipivalathydrochlorid 0,1 % erreicht worden war.

### Ergebnis

Bei lokaler Anwendung am Auge entspricht 0,1 % Epinephrin-$O^1,O^2$-dipivalathydrochlorid hinsichtlich der drucksenkenden Wirkung 1 % Adrenalin. Niedrig dosierte zusätzliche Anwendung von GMS führt nur in Kombination mit Epinephrin-$O^1,O^2$-dipivalathydrochlorid, nicht aber in Kombination mit therapeutisch äquivalenten Mengen Adrenalin zu einer weiteren Senkung des IOP. Die Hyperämie war bei der Kombination Adrenalin + GMS etwa gleich stark ausgeprägt wie bei der Kombination Epinephrin-$O^1,O^2$-dipivalathydrochlorid + GMS. Vier Patienten reagierten auf die Kombination Adrenalin + GMS jedoch mit einer deutlichen Mydriasis, die in drei Fällen zu Sichtbehinderung führte.

### Beispiel 3

Im Anschluß an die Untersuchungen des Beispiels 1 wurden die 10 Patienten der Gruppe 3 zwei Wochen lang mit Adrenalin 0,5 % + GMS 3 % und im Anschluß daran zwei weitere Wochen mit Adrenalin 1 % + GMS 5 % behandelt.

### Ergebnis

| nach Behandlung mit | | durchschnittlicher konstanter Augeninnendruck |
|---|---|---|
| a) Epinephrin-$O^1,O^2$-dipivalathydrochlorid 0,1 % | + GMS 1 % | 2 866 Pa (21,5 mmHg) |
| b) Adrenalin 0,5 % | + GMS 3 % | 3 066 Pa (23 mmHg) |
| c) Adrenalin 1 % | + GMS 5 % | 2 933 Pa (22 mmHg) |

Aus den Untersuchungs-Resultaten geht hervor, daß GMS 1 % in der Kombination mit Epinephrin-$O^1$, $O^2$-dipivalathydrochlorid 0,1 % einen gleichen bis etwas besseren drucksenkenden Effekt am Glaukomauge hat als die Kombination von Adrenalin in therapeutisch äquivalenter Menge (0,5 bis 1 %) mit der drei- bis fünffachen Dosis GMS (3 bis 5 %). Während, wie bei dem Beispiel 1 beschrieben, keiner der Patienten über Nebenwirkungen klagte, gaben bei vorstehendem Beispiel 3 bei b) vier und bei c) sieben Patienten Nebenwirkungen an (auch Mehrfachnennungen), nämlich mäßiges bis starkes Brennen, das bis zu 2 Minuten nach der Anwendung anhielt, Blendempfindlichkeit, gestörtes Nahsehen, stark bitterer Geschmack.

Von den vier Hypertonikern reagierten zwei mit einer Blutdrucksenkung unter den Normalwert ; das Allgemeinbefinden wurde hierdurch nach Angaben der Patienten jedoch nicht beeinträchtigt.

**Ansprüche**

1. Arzneimittel zur lokalen Behandlung des Glaukoms in Form von Augentropfen, Lyophilisat zur Bereitung von Augentropfen, Augensalbe, Augengel und Augeninsert, dadurch gekennzeichnet, daß es 0,05-0,2 % ± Epinephrin-$O^1,O^2$-dipivalat als Salz und 0,05-1,0 % Guanethidin als Salz enthält.

2. Arzneimittel nach Anspruch 1, dadurch gekennzeichnet, daß es ± Epinephrin-$O^1,O^2$-dipivalat als Salz der nachfolgenden Säuren enthält : Salzsäure, Salpetersäure, Schwefelsäure, Borsäure, Phosphorsäure, Zitronensäure, Weinsäure, Essigsäure, Propionsäure oder Bernsteinsäure.

3. Arzneimittel nach Anspruch 1 oder 2, dadurch gekennzeichnet, daß es Guanethidin als Salz der nachfolgenden Säuren enthält : Schwefelsäure, Salzsäure, Salpetersäure, Borsäure, Phosphorsäure, Zitronensäure, Weinsäure, Essigsäure, Propionsäure oder Bernsteinsäure.

4. Arzneimittel nach Anspruch 2, dadurch gekennzeichnet, daß es ± Epinephrin-$O^1,O^2$-dipivalathydrochlorid enthält.

5. Arzneimittel nach Anspruch 3, dadurch gekennzeichnet, daß es Guanethidinsulfat enthält.

6. Arzneimittel nach Anspruch 1 bis 5, dadurch gekennzeichnet, daß es geeignete isotonisierende, stabilisierende, konsistenzgebende, filmbildende und konservierende Hilfsstoffe enthält.

**Claims**

1. A drug for the local treatment of glaucoma in the form of eye drops, lyophilisate for the preparation of eye drops, eye ointment, eye gel and eye insert, characterised in that it contains 0.05-0.2 % ± epinephrine-$O^1,O^2$-dipivalate as a salt and 0.05-1.0 % of guanethidine as a salt.

2. A drug as claimed in claim 1, characterised in that it contains ± epinephrine-$O^1,O^2$-dipivalate as a salt of the following acids : hydrochloric acid, nitric acid, sulphuric acid, boric acid, phosphoric acid, citric acid, tartaric acid, acetic acid, propionic acid or succinic acid.

3. A drug as claimed in claim 1 or 2, characterised in that it contains guanethidine as a salt of the following acids : sulphuric acid, hydrochloric acid, nitric acid, boric acid, phosphoric acid, citric acid, tartaric acid, acetic acid, propionic acid or succinic acid.

4. A drug as claimed in claim 2, characterised in that it contains ± epinephrine-$O^1,O^2$-dipivalatehydrochloride.

5. A drug as claimed in claim 3, characterised in that it contains guanethidine sulphate.

6. A drug as claimed in claims 1 to 5, characterised in that it contains suitable isotonicizing, stabilizing, consistency-giving, film-forming and preserving auxiliary substances.

**Revendications**

1. Médicament pour le traitement local du glaucome sous la forme de gouttes oculaires, de lyophilisat pour la préparation de ces gouttes, de pommade, de gel et d'insert oculaires, caractérisé en ce qu'il contient 0,05-0,2 % de ± épinéphrine-$O^1,O^2$-dipivalate sous forme saline et 0,05-1,0 % de guanéthidine sous forme saline.

2. Médicament selon la revendication 1, caractérisé en ce qu'il contient du ± épinéphrine-$O^1,O^2$-dipivalate sous la forme d'un sel des acides suivants : chlorhydrique, nitrique, sulfurique, borique, phosphorique, citrique, tartrique, acétique, propionique ou succinique.

3. Médicament selon la revendication 1 ou 2 caractérisé en ce qu'il contient de la guanéthidine sous forme de sels des acides suivants : sulfurique, chlorhydrique, nitrique, borique, phosphorique, citrique, tartrique, acétique, propionique ou succinique.

4. Médicament selon la revendication 2, caractérisé en ce qu'il contient du chlorhydrate de ± épinéphrine-$O^1,O^2$-dipivalate.

5. Médicament selon la revendication 3, caractérisé en ce qu'il contient du sulfate de guanéthidine.

6. Médicament selon les revendications 1 à 5, caractérisé en ce qu'il contient des substances auxiliaires isotonifiantes, stabilisantes et filmogènes, et des substances auxiliaires aptes à assurer sa conservation.